# FASCICULE DE BREVET EUROPEEN

(11) **EP 3 821 397 B1**
(45) Date de publication et mention de la délivrance du brevet: **06.09.2023**
(21) Numéro de dépôt: 19736406.0
(22) Date de dépôt: 10.07.2019
(51) Int. Cl.: G06T 7/11

(54) **PROCEDE DE DECOUPAGE D'UN MODELE D'UNE ARCADE DENTAIRE**
VERFAHREN ZUM SCHNEIDEN EINES MODELLS EINES ZAHNBOGENS
METHOD FOR CUTTING A MODEL OF A DENTAL ARCH

(30) Priorité: 13.07.2018 FR 1856502
(43) Date de publication de la demande: 19.05.2021
(73) Titulaire: Dental Monitoring, 75017 Paris (FR)
(72) Inventeur: SALAH, Philippe, 75020 PARIS (FR); PELLISSARD, Thomas, 75004 PARIS (FR); GHYSELINCK, Guillaume, 56169 CANTIN (FR); DEBRAUX, Laurent, 75020 PARIS (FR)
(74) Mandataire: Cabinet Nony
(86) Numéro de dépôt international: PCT/EP2019/068564
(87) Numéro de publication internationale: WO 2020/011866

(56) Documents cités:
- US-A1- 2018 028 294
- ZHIGE XIE ET AL: "Projective Feature Learning for 3D Shapes with Multi-View Depth Images", COMPUTER GRAPHICS FORUM, vol. 34, no. 7, 1 octobre 2015 (2015-10-01), pages 1-11, XP055516937, GB ISSN: 0167-7055, DOI: 10.1111/cgf.12740
- Evangelos Kalogerakis ET AL: "3D Shape Segmentation with Projective Convolutional Networks", , 13 novembre 2017 (2017-11-13), XP055516944, Extrait de l'Internet: URL:https://arxiv.org/pdf/1612.02808 [extrait le 2018-10-18]
- YUNHAI WANG ET AL: "Projective analysis for 3D shape segmentation", ACM TRANSACTIONS ON GRAPHICS (TOG), ACM, US, vol. 32, no. 6, 1 novembre 2013 (2013-11-01), pages 1-12, XP058033888, ISSN: 0730-0301, DOI: 10.1145/2508363.2508393
- Evan Shelhamer ET AL: "Fully Convolutional Networks for Semantic Segmentation", , 20 mai 2016 (2016-05-20), XP055516953, Extrait de l'Internet: URL:https://arxiv.org/pdf/1605.06211 [extrait le 2018-10-18]
- GUO KAN ET AL: "Image-guided 3D model labeling via multiview alignment", GRAPHICAL MODELS, ELSEVIER, SAN DIEGO, CA, US, vol. 96, 10 février 2018 (2018-02-10), pages 30-37, XP085360436, ISSN: 1524-0703, DOI: 10.1016/J.GMOD.2018.02.001
- XIAOJIE XU ET AL: "3D Tooth Segmentation and Labeling using Deep Convolutional Neural Networks", IEEE TRANSACTIONS ON VISUALIZATION AND COMPUTER GRAPHICS., 22 mai 2018 (2018-05-22), pages 1-13, XP055516965, US ISSN: 1077-2626, DOI: 10.1109/TVCG.2018.2839685

## Description

### Domaine technique

La présente invention concerne un procédé de découpage d'un modèle tridimensionnel d'une arcade dentaire.

### Etat de la technique

Le découpage d'un modèle tridimensionnel en modèles élémentaires est une opération par laquelle le modèle est découpé afin de délimiter la représentation d'un ou plusieurs éléments de la scène modélisée. En particulier, le Demandeur a inventé plusieurs procédés dans lesquels un modèle d'une arcade dentaire est découpé afin de définir des modèles élémentaires pour chaque dent, ou « modèles de dent ». Le modèle ainsi découpé peut être ensuite déformé, par déplacement des modèles de dent, pour simuler l'effet d'un traitement orthodontique ou l'évolution d'une récidive, ou un traitement esthétique.

Le découpage peut être partiellement automatisé, en particulier pour définir le contour des dents. L'intervention d'un opérateur reste cependant nécessaire pour affiner et valider le découpage, notamment pour tenir compte du contexte,

Dans ce domaine, l'article "3D Shape Segmentation with Projective Convolutional Networks" par Kalogerakis et al. divulgue une méthode de segmentation de modèle 3D basée sur le traitement par réseau de neurones d'images 2D projetées du modèle 3D.

Il existe un besoin permanent pour accélérer l'opération de découpage d'un modèle d'une arcade dentaire.

Un but de l'invention est de répondre à ce besoin.

### Résumé de l'invention

L'invention propose un procédé de découpage d'un modèle tridimensionnel d'une scène ou « modèle de scène », en particulier d'une scène dentaire, ledit procédé étant défini à la revendication 1.

Comme on le verra plus en détail dans la suite de la description, un procédé de découpage selon l'invention utilise les capacités d'un réseau de neurones pour l'analyse d'images, en l'occurrence de vues du modèle de scène, pour enrichir la description de ce modèle. Le procédé peut être en particulier utilisé pour identifier, dans le modèle de scène, les régions dont la nature a été identifiée, par le réseau de neurones, sur la vue d'analyse.

De préférence, la zone élémentaire définit la représentation d'un élément physique de la scène, par exemple une dent. La zone élémentaire est alors qualifiée de « zone de dent ».

L'attribut de la zone élémentaire peut être en particulier choisi parmi un numéro de dent, un type de dent, un paramètre de forme de la dent, un indice de déflexion en mésial et distal du bord incisif, un niveau d'abrasion, un paramètre d'apparence de la dent, un paramètre relatif à l'état de la dent ou relatif à un appareil dentaire porté par la dent.

L'attribut de modèle peut être identique ou différent de l'attribut de la zone élémentaire. De préférence, ces deux attributs sont identiques, et en particulier peuvent être un numéro de la dent.

Le procédé comporte encore, après l'étape c), l'étape suivante :
d) modification de la vue d'analyse, puis reprise à l'étape b).

En multipliant les cycles d'étapes b) à d), il est ainsi possible d'explorer le modèle de scène, c'est-à-dire d'acquérir de nombreuses vues d'analyse, dans des conditions d'observations différentes, et en particulier sous des orientations différentes, afin de parvenir à affecter des valeurs, pour ledit attribut de modèle, à tous les voxels dudit modèle de scène. De préférence, le procédé comporte encore, après plus de 10, plus de 100, ou plus de 1000 cycles d'étapes b) à d), l'étape e) suivante :
e) regroupement des régions du modèle de scène qui ont la même valeur pour ledit attribut de modèle, de manière à créer un modèle élémentaire.

Par exemple, le modèle de scène peut être un modèle d'une arcade dentaire, ou « modèle d'arcade », et l'attribut de la zone élémentaire peut être un numéro de dent. Les cycles d'étapes b) à d) permettent alors, en observant selon différentes directions la partie du modèle d'arcade qui représente une dent, d'identifier progressivement tous les voxels du modèle d'arcade qui appartiennent à la représentation de cette dent, et ainsi de définir, sans intervention d'un opérateur, un modèle de dent.

De préférence, le procédé comporte encore, après l'étape e), l'étape suivante :
f) déplacement et/ou déformation et/ou suppression du modèle élémentaire. Le déplacement des modèles élémentaires permet en particulier de simuler une situation dentaire, notamment orthodontique, passée ou future.

Dans un mode de réalisation, le modèle de scène est constitué d'un assemblage de régions de base, classiquement triangulaires et, quand, à l'étape c), une valeur est affectée, à un attribut du modèle de scène, pour une région qui inclut au moins un voxel d'une région de base, on affecte ladite valeur à toute ladite région de base.

Par exemple, si on identifie, à l'étape c), qu'un voxel est la représentation d'une dent n°4, la région triangulaire qui inclut ce voxel est immédiatement considérée comme représentant la dent n°4. Le découpage du modèle de scène en est avantageusement considérablement accéléré.

Le procédé comporte les caractéristiques suivantes :
- la vue d'analyse acquise à l'étape a) et/ou une ou plusieurs des vues d'analyse modifiées à l'étape d), de préférence la vue d'analyse acquise à l'étape a) et toutes les vues d'analyse modifiées à l'étape d), sont des vues d'analyse de base obtenues suivant les étapes suivantes :
   1) détermination d'une orientation prédéterminée du modèle de scène, dite « orientation de base » ;
   2) détermination d'un ensemble de différentes conditions d'observation du modèle de scène positionné dans ladite orientation de base, dites « conditions d'observation de base » ;
   3) acquisition d'un ensemble de vues d'analyse de base, chaque vue d'analyse de base étant acquise dans des conditions d'observation de base respectives ;
- à l'étape 2), on détermine l'ensemble desdites conditions d'observation de base de manière à minimiser le nombre desdites conditions d'observation de base, et à maintenir à une valeur supérieure à un seuil prédéterminé, pour chacun des modèles de scène de base d'un groupe de modèles de scène de base, le rapport de la surface totale de l'ensemble des modèles élémentaires déterminés à l'étape e) sur la surface du modèle de scène ;
De manière optionnelle , le procédé comporte une ou plusieurs des caractéristiques suivantes:
- le groupe de modèles de scène de base comporte plus de 10, de préférence plus de 100, de préférence plus de 1 000 modèles de scène de base, chaque modèle de scène de base modélisant au moins une partie d'une arcade d'un patient respectif ;
- le procédé comporte, à l'issue de l'étape e), l'étape consistant à affecter chacun d'au moins une partie des voxels orphelins du modèle de scène, de préférence de chacun des voxels orphelins, aux modèles élémentaires créés à l'étape e), un voxel orphelin ne pouvant être affecté à plus d'un modèle élémentaire, un voxel orphelin étant un voxel non regroupé au sein d'un modèle élémentaire lors de l'étape e).

Un procédé selon l'invention est au moins en partie, de préférence entièrement mis en oeuvre par ordinateur.

### Définitions

Un « patient » est une personne pour laquelle un procédé selon l'invention est mis en oeuvre, indépendamment du fait que cette personne suive un traitement orthodontique ou non.

Le "plan d'occlusion" est le plan qui fournit la meilleure corrélation linéaire avec l'ensemble des points de contact entre les dents de l'arcade maxillaire d'une part et les dents de l'arcade mandibulaire d'autre part.

Le "plan longitudinal médian" est le plan sensiblement vertical lorsque le patient tient la tête droite, qui sépare de manière sensiblement symétrique des parties droite et gauche de chaque arcade. L'axe longitudinal médian est l'axe qui s'étend à l'intersection du plan d'occlusion et du plan longitudinal médian.

Une « situation dentaire » définit un ensemble de caractéristiques relatives à une arcade d'un patient à un instant, par exemple la position des dents, leur forme, la position d'un appareil orthodontique, etc. à cet instant.

Par « modèle », on entend un modèle tridimensionnel numérique. Un modèle est constitué d'un ensemble de voxels. Un « modèle d'une arcade » est un modèle représentant au moins une partie d'une arcade dentaire, de préférence au moins 2, de préférence au moins 3, de préférence au moins 4 dents.

Dans un souci de clarté, on distingue classiquement le « découpage » d'un modèle de scène en « modèles élémentaires » et la « segmentation » d'une image, en particulier d'une vue d'un modèle de scène, en « zones élémentaires ». Les modèles élémentaires et les zones élémentaires sont des représentations, en 3D ou en 2D respectivement, d'un élément d'une scène réelle, par exemple d'une dent.

Une observation d'un modèle, dans des conditions d'observations déterminées, en particulier selon un angle et à une distance déterminés, est appelée une « vue ».

Une « image » est une représentation, en deux dimensions et formée de pixels, d'une scène. Une vue est un exemple d'image.

Une « scène » est constituée par un ensemble d'éléments qui peuvent être observés simultanément. Une « scène dentaire » est une scène comportant au moins une partie d'une arcade dentaire.

Un attribut de dent est un attribut dont la valeur est spécifique aux dents. De préférence, une valeur d'un attribut de dent est affectée à chaque zone de dent de la vue considérée ou à chaque modèle de dent d'un modèle d'arcade dentaire considéré. Un attribut de dent ne concerne pas la vue ou le modèle dans son ensemble. Il tient sa valeur du fait de caractéristiques de la dent à laquelle il se réfère.

Par « vue d'une arcade », « représentation d'une arcade », « scan d'une arcade », ou « modèle d'une arcade », on entend une vue, une représentation, un scan ou un modèle de tout ou partie de ladite arcade dentaire.

Une « base d'apprentissage » est une base d'enregistrements informatiques adaptée à l'entrainement d'un réseau de neurones. Chaque enregistrement comporte un objet, par exemple une image, et des informations sur cet objet, ou « descriptif ». Un descriptif comporte des valeurs pour des attributs de l'objet. Par exemple, un attribut d'une image d'une scène dentaire peut servir à identifier les numéros des dents représentées. L'attribut est alors « numéro de dent » et pour chaque dent, la valeur de cet attribut est le numéro de cette dent.

Un « réseau de neurones » ou « réseau neuronal artificiel » est un ensemble d'algorithmes d'intelligence artificielle bien connu de l'homme de l'art.

Dans la présente description, les qualificatifs « historique » ou « d'analyse » sont utilisés à des fins de clarté.

Dans un souci de clarté, on qualifie de « zones » et de « régions » les surfaces d'une image et d'un modèle, respectivement. On distingue également un « descriptif », qui décrit l'objet d'un enregistrement d'une base d'apprentissage et qui, en particulier, comporte des valeurs d'attribut de cet objet, et une « description » qui décrit un modèle. Un « descriptif élémentaire » décrit spécifiquement une zone élémentaire. Une « description élémentaire » décrit spécifiquement un modèle élémentaire.

Il faut interpréter "comprenant " ou "comportant " ou "présentant " de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente, schématiquement, les différentes étapes d'un procédé selon l'invention ;
- la figure 2 représente un exemple d'un modèle d'une arcade dentaire, c'est-à-dire d'un modèle de scène dentaire ;
- la figure 3 représente un exemple d'une « vue d'analyse » d'un modèle d'une arcade dentaire ;
- la figure 4 représente les régions dudit modèle d'arcade dentaire qui sont « parentes » des zones élémentaires identifiées sur la vue d'analyse représentée sur la figure 3 ;
- la figure 5 représente, schématiquement, les différentes étapes d'un procédé pour créer un modèle de scène dentaire par assemblage de modèles de dent de base ;
- les figures 6A-6D illustrent le traitement effectué pour déterminer le plan d'occlusion du modèle de scène dentaire de la figure 2 ;
- la figure 7 illustre un exemple d'un ensemble de conditions d'observation de base d'un modèle de scène dentaire ;
- la figure 8 illustre un exemple d'un ensemble de vues d'analyse de base d'un modèle de scène dentaire, cet ensemble étant obtenu à partir d'une configuration d'observation regroupant 16 conditions d'observation de base.

### Description détaillée

La description détaillée qui suit est celle de modes de réalisation préférés, mais n'est pas limitative.

### Création de la base d'apprentissage et entrainement du réseau de neurones

Préalablement à l'étape b) et de préférence à l'étape a), un réseau de neurones doit être entrainé avec une base d'apprentissage.

La base d'apprentissage peut être créée suivant des méthodes conventionnelles.

Classiquement, la base d'apprentissage est constituée de plus de 1 000 enregistrements « historiques », chaque enregistrement comportant :
- une vue « historique » d'un modèle tridimensionnel numérique « historique » modélisant une scène « historique », en particulier une arcade dentaire d'un patient « historique » ;
- un descriptif « historique » de la vue historique, un descriptif historique comportant une valeur pour au moins un attribut d'une zone élémentaire « historique » représentant, dans la vue historique, un élément de la scène historique.

Un modèle historique peut être préparé à partir de mesures effectuées sur les dents du patient historique ou sur un moulage de ses dents, par exemple un moulage en plâtre.

Le modèle historique est de préférence obtenu à partir d'une situation réelle, de préférence créé avec un scanner 3D.

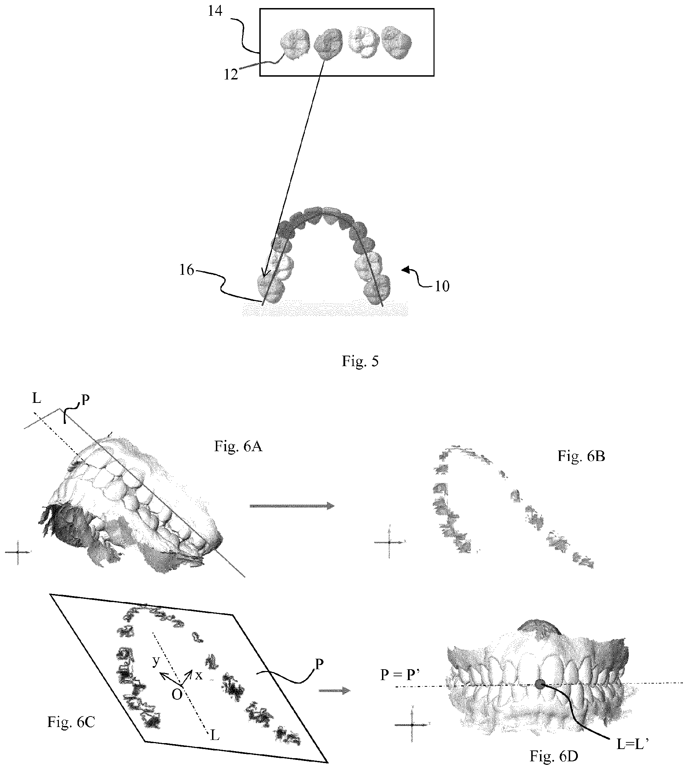

Dans un mode de réalisation, le modèle historique est théorique, c'est-à-dire ne correspond pas à une situation réelle. En particulier, le modèle historique 10 (voir figure 5) peut être créé par assemblage d'un ensemble de modèles de dents de base 12 choisis dans une bibliothèque numérique 14. L'agencement des modèles de dent de base est déterminé pour que le modèle historique soit réaliste, c'est-à-dire corresponde à une situation qui aurait pu se rencontrer chez un patient. En particulier, les modèles de dents de base sont disposés suivant un arc 16, en fonction de leur nature, et orientés de manière réaliste.

Un modèle historique peut être observé dans différentes conditions d'observations pour acquérir différentes vues historiques.

Chaque vue historique représente un ensemble de zones élémentaires historiques qui, chacune, représentent un élément de la scène modélisée par le modèle historique. Cet élément peut être une dent.

Par exemple, chaque enregistrement de la base d'apprentissage peut comporter une vue historique d'une arcade dentaire et un descriptif contenant pour chaque zone de dent, c'est-à-dire pour chaque zone élémentaire représentant une dent sur la vue historique, une valeur pour au moins un attribut de dent.

L'attribut de dent est de préférence choisi parmi un numéro de dent, un type de dent, un paramètre de forme de la dent, par exemple une largeur de dent, en particulier une largeur mésio-palatine, une épaisseur, une hauteur de couronne, un indice de déflexion en mésial et distal du bord incisif, ou un niveau d'abrasion, un paramètre d'apparence de la dent, en particulier un indice de translucidité ou un paramètre de couleur, ou un paramètre relatif à l'état de la dent, par exemple « abrasée », « cassée », « cariée » ou « appareillée » (c'est-à-dire en contact avec un appareil dentaire, par exemple orthodontique).

Par exemple, l'attribut de dent « type de dent » aura la valeur « incisive », « canine » ou « molaire » selon que la zone élémentaire représente une incisive, une canine ou une molaire, respectivement. L'attribut de dent « situation pathologique » aura la valeur « dent saine », « dent cassée », « dent usée » ou « dent cariée », par exemple.

L'attribut de dent peut être également relatif à une interaction entre un appareil dentaire et la dent considérée. L'attribut de dent peut être par exemple « collage sur la dent » et prendre la valeur « collé » ou « décollé ».

Une vue historique peut également représenter des zones élémentaires historiques qui, chacune, représentent un élément différent d'une dent. Notamment, on peut définir, à partir du modèle historique, d'autres zones élémentaires que les zones de dent, et notamment des modèles pour la langue, et/ou la bouche, et/ou les lèvres, et/ou les mâchoires, et/ou la gencive, et/ou un appareil dentaire, de préférence orthodontique, et leur affecter des valeurs pour des attributs de langue, et/ou de bouche, et/ou de lèvres, et/ou de mâchoires, et/ou de gencive, et/ou d'appareil dentaire, respectivement.

Un attribut de langue peut être, par exemple, relatif à la position de la langue (par exemple prendre la valeur « en retrait »).

Un attribut de bouche peut être, par exemple, relatif à l'ouverture de la bouche du patient (par exemple prendre la valeur « bouche ouverte » ou « bouche fermée »).

Un attribut d'appareil dentaire peut être, par exemple, relatif à la présence d'un appareil dentaire et/ou relatif à son état (par exemple prendre la valeur « appareil intact », « appareil cassé » ou « appareil endommagé »).

Classiquement, les descriptifs historiques sont générés par un opérateur qui délimite, au moyen d'un ordinateur, les zones élémentaires, de préférence au moins les zones de dent, et qui, après avoir identifié une valeur d'au moins un attribut d'une zone élémentaire, par exemple la nature d'une dent représentée, par exemple « canine supérieure droite », affecte cette valeur audit attribut. Cette opération est appelée « labelling ».

La génération d'un descriptif historique peut être également, au moins en partie, automatique.

En particulier, de préférence, on génère une description du modèle historique, puis on crée, au moins en partie, le descriptif historique à partir de la description du modèle historique.

A cet effet, de préférence, le modèle historique est découpé en modèles élémentaires historiques, puis on génère, dans la description du modèle historique, une description élémentaire spécifique pour chaque modèle élémentaire historique.

Dans un mode de réalisation préféré, la forme d'un modèle élémentaire historique, par exemple d'un modèle de dent particulier, est analysée au moyen d'un réseau de neurones entrainé pour définir une valeur pour au moins un attribut, par exemple un numéro de dent.

La description d'un modèle historique comporte ainsi un ensemble de données relatives aux modèles élémentaires historiques, par exemple aux modèles élémentaires historiques qui modélisent les dents, et de préférence des données relatives au modèle historique dans son ensemble.

On peut ensuite inclure, dans le descriptif historique d'une vue historique, un descriptif élémentaire historique pour chaque représentation d'un modèle élémentaire historique sur la vue historique, au moins une partie du descriptif élémentaire historique étant héritée de la description élémentaire historique spécifique au modèle élémentaire historique.

Un modèle élémentaire historique peut en particulier modéliser une dent, et un ou plusieurs attributs de dent peuvent être associés à ce modèle élémentaire historique. Ces attributs de dents peuvent être en particulier choisis parmi les attributs de dents listés ci-dessus pour les zones de dent.

Par exemple, on crée des modèles élémentaires historiques représentant les dents, ou « modèles de dent historiques », dans le modèle historique, et, dans la description du modèle historique, on crée une description élémentaire historique spécifique pour chaque modèle de dent historique, par exemple pour identifier les numéros de dent correspondants. On peut alors facilement renseigner en conséquence le descriptif historique, et en particulier les descriptifs élémentaires historiques. Notamment, les numéros des dents des modèles de dent historiques peuvent être affectés aux représentations de ces modèles de dents historiques sur la vue historique. Avantageusement, une fois que le modèle historique et sa description ont été créés, il est ainsi possible de générer des enregistrements historiques par ordinateur, sans intervention humaine. La création du descriptif historique peut donc être, au moins partiellement, automatisée. Le risque d'erreur en est avantageusement limité.

En outre, ce procédé permet avantageusement, en modifiant la vue historique d'un même modèle historique, de générer un grand nombre d'enregistrements historiques. De préférence, après avoir généré un enregistrement historique, on modifie la vue historique du même modèle historique, puis on crée un nouvel enregistrement historique.

Dans un mode de réalisation, le modèle historique est déformé, par exemple par déplacement d'un ou plusieurs modèles de dent, puis on génère de nouveaux enregistrements historiques à partir de vues historiques du modèle historique déformé. Avantageusement, il devient ainsi possible de créer différents modèles historiques qui ne sont pas exclusivement issus de mesures sur un patient, et en particulier d'un scan de l'arcade dentaire du patient. Les modèles historiques peuvent être en particulier créés pour simuler des situations dentaires pour lesquelles peu de données réelles sont disponibles, par exemple relatives à des pathologies rares.

Le réseau de neurones peut être en particulier choisi parmi :
- les réseaux spécialisés dans la classification d'images, appelés « CNN » (« Convolutional neural network »), par exemple
   - AlexNet (2012)
   - ZF Net (2013)
   - VGG Net (2014)
   - GoogleNet (2015)
   - Microsoft ResNet (2015)
   - Caffe: BAIR Reference CaffeNet, BAIR AlexNet
   - Torch:VGG_CNN_S,VGG_CNN_M,VGG_CNN_M_2048,VGG_CNN_M_10 24,VGG_CNN_M_128,VGG_CNN_F,VGG ILSVRC-2014 16-layer,VGG ILSVRC-2014 19-layer,Network-in-Network (Imagenet & CIFAR-10)
   - Google : Inception (V3, V4) ;
- les réseaux spécialisés dans la localisation, et détection d'objets dans une image, les Object Détection Network, par exemple :
   - R-CNN (2013)
   - SSD (Single Shot MultiBox Detector : Object Détection network), Faster R-CNN (Faster Region-based Convolutional Network method : Object Détection network)
   - Faster R-CNN (2015)
   - SSD (2015).

La liste ci-dessus n'est pas limitative.

Pour être opérationnel, le réseau de neurones doit être entrainé par un processus d'apprentissage appelé *« deep learning »,* à partir de la base d'apprentissage.

En présentant les enregistrements de la base d'apprentissage en entrée du réseau de neurones, ce dernier apprend progressivement comment segmenter une vue d'analyse qu'on lui présente, c'est-à-dire comment l'analyser et générer un descriptif permettant d'identifier et de qualifier des zones élémentaires de cette vue. En particulier, le réseau de neurones apprend à identifier, sur une vue d'analyse d'une arcade dentaire, les représentations des dents et à leur attribuer des valeurs d'attribut correspondantes.

La qualité de l'analyse réalisée par le réseau de neurones entrainé avec la base d'apprentissage dépend directement du nombre d'enregistrements de la base d'apprentissage. De préférence, la base d'apprentissage comporte plus de 5 000, de préférence plus de 10 000, de préférence plus de 30 000, de préférence plus de 50 000, de préférence plus de 100 000 enregistrements. Plus le nombre d'enregistrements est élevé, meilleure est la capacité d'analyse du réseau de neurones.

### Découpage d'un modèle

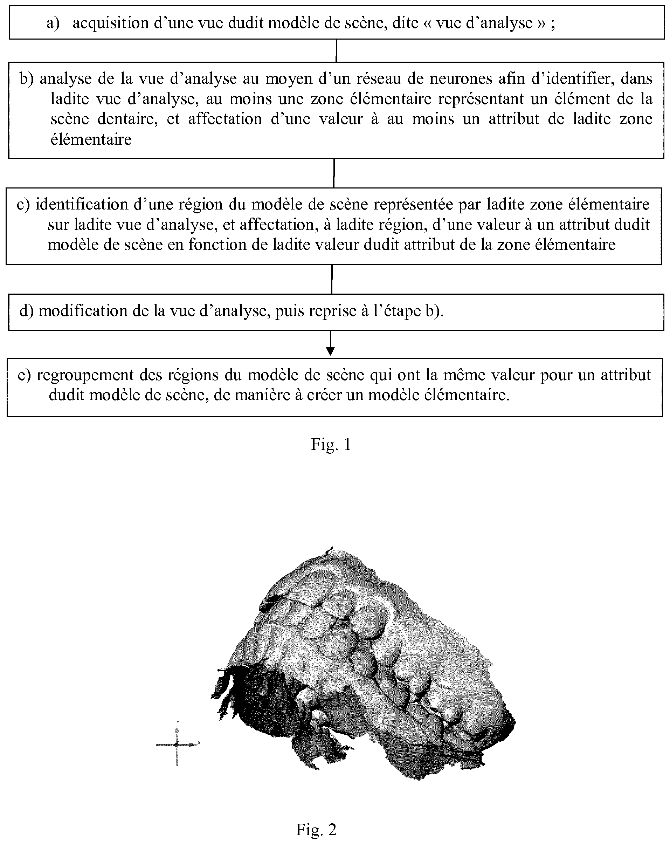

Le modèle de scène modélise une scène et en particulier peut modéliser une scène dentaire d'un patient (figure 2). Il peut être réalisé suivant les techniques décrites ci-dessus pour la génération d'un modèle historique, et en particulier par un scan d'une arcade d'un patient.

Pour découper un modèle de scène, on procède, après avoir créé la base d'apprentissage et entrainé le réseau de neurones, suivant les étapes a) à c) (figure 1).

**A l'étape a),** on acquiert une vue du modèle de scène, ou « vue d'analyse ». La vue d'analyse représente donc le modèle de scène tel qu'observé dans des conditions d'observations déterminées. Par exemple une vue d'analyse peut représenter le modèle de scène vu de face. La vue d'analyse contient des zones élémentaires qui chacune représente un élément de la scène modélisée. Par exemple, si la vue d'analyse est une vue de face, elle peut comporter une représentation pour l'incisive droite, une représentation pour l'incisive gauche, et une représentation pour la gencive. Ces représentations constituent chacune une zone élémentaire.

**A l'étape b),** on présente la vue d'analyse au réseau de neurones entrainé. Le réseau de neurones analyse la vue d'analyse et détermine des valeurs pour des attributs des zones élémentaires. Ainsi, le réseau de neurones détermine-t-il un descriptif pour la vue d'analyse. En particulier, il peut déterminer, avec une probabilité, les contours des zones élémentaires et la nature des éléments représentés par ces zones élémentaires. Par exemple, il peut déterminer qu'une zone élémentaire au centre de la vue d'analyse est une incisive avec une probabilité de 95%. Des essais ont montré qu'un réseau de neurones est bien adapté pour analyser une vue d'un modèle dentaire.

**A l'étape c),** on identifie, pour chaque zone élémentaire déterminée à l'étape b), la région du modèle de scène dont elle est issue.

Une vue d'un modèle d'arcade est toujours unique, c'est-à-dire qu'elle ne peut être acquise que dans des conditions d'observation spécifiques. A une vue d'analyse correspondent donc, de manière bijective, des conditions d'observations particulières. Les conditions d'observations ayant permis d'acquérir la vue d'analyse étant connues, il est donc possible de déduire, pour chaque zone élémentaire, une et une seule région du modèle de scène, dite « région parente », qui, lorsqu'elle est observée dans les conditions d'observations, est représentée par la zone élémentaire.

Des valeurs du descriptif de la vue d'analyse, et en particulier les valeurs d'attribut qui sont spécifiques à la zone élémentaire, peuvent dès lors être affectées à la région parente.

Par exemple, si une valeur d'attribut d'une zone élémentaire indique que « la zone élémentaire est une représentation au moins partielle d'une incisive », le voxel du modèle de scène qui est représenté, sur la vue d'analyse, par un pixel appartenant à la zone élémentaire peut hériter de cette valeur d'attribut, et être considéré comme appartenant également « à la représentation d'une incisive », c'est-à-dire comme appartenant à un modèle élémentaire d'une incisive. On peut aussi dire que le voxel est « affecté » au modèle élémentaire représentant l'incisive.

La résolution du modèle de scène étant limitée, ce modèle est classiquement un assemblage de régions de base de forme triangulaire, ou « triangles », de différentes tailles. Dans la mesure où une valeur d'attribut est affectée à une région du modèle de scène qui contient au moins un voxel d'un triangle, ladite région peut être étendue de manière à inclure tout le triangle. Le découpage du modèle de scène en est accéléré.

A l'issue de l'étape c), sensiblement seules les régions parentes de zones élémentaires de la vue d'analyse ont une description précisant une valeur d'attribut déduite de l'analyse de la vue d'analyse.

**A l'étape d),** on modifie donc la vue d'analyse, c'est-à-dire on modifie les conditions d'observation du modèle de scène, puis on recommence les étapes b) et c). La vue d'analyse ayant été modifiée, il en est de même des zones élémentaires et des régions parentes correspondantes.

Le cycle des étapes b) à d) est de préférence repris, par exemple plus de 10, plus de 100, ou plus de 1000 fois, de préférence en tournant autour du modèle de scène. De préférence, le cycle des étapes b) à d) est répété jusqu'à ce que l'ensemble des régions parentes identifiées et ayant hérité de valeurs d'attribut de zones élémentaires représente plus de 50%, de préférence plus de 60%, plus de 70%, plus de 80%, plus de 90%, de préférence sensiblement 100% de la surface du modèle de scène.

A l'issue de l'étape d), les régions parentes qui partagent une même valeur pour un attribut, par exemple les régions qui sont toutes considérées comme « appartenant à l'incisive supérieure droite », peuvent être regroupées pour former un modèle élémentaire, par exemple un modèle élémentaire d'une incisive supérieure droite.

Le procédé permet ainsi de découper un modèle de scène, et en particulier de découper un modèle d'arcade afin de créer des modèles élémentaires, notamment pour les dents du modèle d'arcade.

Le modèle de scène découpé peut être utilisé dans toutes les applications dans lesquelles des modèles de scène découpés sont utilisés à ce jour. Notamment, le modèle de scène découpé peut être utilisé pour simuler in traitement orthodontique ou pour concevoir un appareil orthodontique, à des fins thérapeutiques ou non, par exemple à des fins de recherche ou à de fins purement esthétiques.

### Exemple

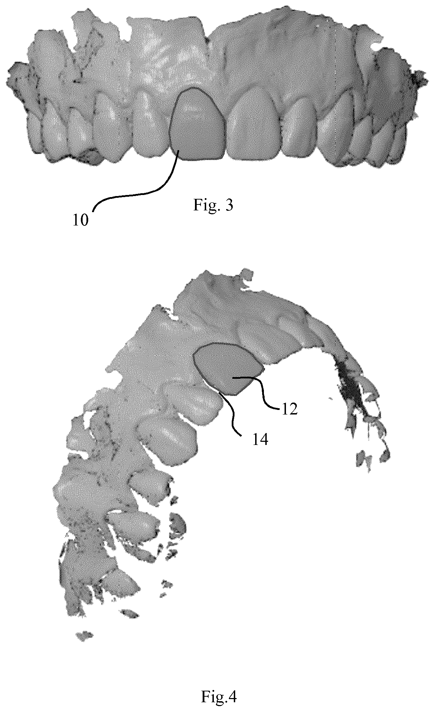

La figure 3 est une vue d'analyse d'une arcade dentaire. Le réseau de neurones a permis d'identifier les représentations, sur cette vue, de différents éléments de la scène dentaire, et en particulier a permis d'identifier des zones élémentaires qui sont des représentations de dents et de la gencive. Notamment, il a identifié que la zone élémentaire 10 est la représentation d'une incisive. La valeur de l'attribut « type de dent » de la zone élémentaire 10 est donc « incisive ».

A l'étape c), les régions parentes des différentes zones élémentaires ont été identifiées dans le modèle représenté sur la vue d'analyse. La figure 4 représente ces différentes régions parentes. En particulier, la région 12 est parente de la zone élémentaire 10. Il est donc possible de conclure que la région 12 du modèle est une région qui représente, au moins partiellement, une incisive. La valeur de l'attribut de modèle « type de dent » de la région 12 est donc « incisive ».

On observe que la figure 4 est incomplète. En effet, les parties du modèle qui ne sont pas visibles sur la vue d'analyse n'ont pu être identifiées. Par exemple, les parties qui s'étendent entre l'incisive et la canine adjacente n'ont pas été identifiées, ce qui laisse un espace vide 14 sur la figure 4.

Pour identifier la nature de ces parties, par exemple pour déterminer, dans l'espace vide 14, les surfaces de modèle qui appartiennent à la gencive, à l'incisive ou à la canine adjacente, une ou plusieurs autres vues d'analyse, représentant la partie du modèle correspondant à l'espace vide 14, doivent être acquises, puis soumises au réseau de neurones.

Le cyclage entre les étapes b) et d) permet ainsi d'identifier toutes les surfaces de l'incisive qui sont représentées sur le modèle. Le modèle peut être ainsi découpé de manière automatique.

### Perfectionnement

Pour découper précisément un modèle de scène en modèles élémentaires, il est préférable de multiplier les cycles des étapes b) à d), de préférence d'effectuer plus de 1000 cycles, de préférence en tournant autour du modèle de scène. Cette procédure est longue à mettre en oeuvre. Il existe un besoin pour l'accélérer.

En poursuivant leurs recherches, les inventeurs ont découvert une solution pour répondre à ce besoin, sans nuire à la précision. Plus précisément, ils proposent un procédé de découpage « perfectionné » comportant les étapes suivantes :
1) détermination d'une orientation du modèle de scène dans l'espace, dite « orientation de base » ;
2) détermination d'un ensemble de différentes conditions d'observation du modèle de scène positionné dans ladite orientation de base, dites « conditions d'observation de base » ;
3) acquisition d'un ensemble de vues d'analyse, dites « vues d'analyse de base », chaque vue d'analyse de base étant acquise dans des conditions d'observation de base respectives ;
4) mise en oeuvre du procédé de découpage comportant les étapes a) à e) pour chacune des vues d'analyse de base.

Autrement dit, à l'étape 4), les vues d'analyse analysées lors des différentes occurrences de l'étape b), c'est-à-dire la vue d'analyse issue de l'étape a), puis les vues d'analyse modifiées issues des étapes d) successives, sont à chaque fois constituées par une vue d'analyse de base différente, le procédé de découpage selon l'invention étant mis en oeuvre autant de fois que nécessaire pour que toutes les vues d'analyse de base aient été utilisées.

Comme on le verra plus en détail dans la suite de la description, l'orientation du modèle de scène permet de limiter considérablement le nombre de cycles, tout en conservant une bonne résolution des modèles élémentaires. De manière inattendue, avec des modèles de scène représentant des modèles d'arcades dentaires, les inventeurs sont parvenus, avec moins de 20 vues d'analyse de base, à un degré de précision supérieur à 95%.

**A l'étape 1),** le modèle de scène est une scène dentaire d'une arcade dentaire ou des deux arcades dentaires d'un patient.

De préférence, comme représenté sur la figure 6D, le modèle de scène est orienté dans l'espace de manière à confondre
- le plan d'occlusion P du modèle de scène avec un plan de référence P' prédéterminé, et
- l'axe longitudinal médian L du modèle de scène qui s'étend dans le plan d'occlusion P dudit modèle, avec un axe de référence L' prédéterminé qui s'étend dans le plan de référence.

Le plan d'occlusion et l'axe longitudinal médian du modèle de scène peuvent être déterminés manuellement, de manière approximative. De préférence, ils sont déterminés par traitement informatique.

De préférence, le modèle de scène est un modèle des arcades bouche fermée, c'est-à-dire dans une position dans laquelle des dents de l'arcade maxillaire sont en contact avec des dents de l'arcade mandibulaire (figure 6A).

Classiquement, le modèle de scène dentaire fourni par un scanner tridimensionnel permet de distinguer l'arcade maxillaire de l'arcade mandibulaire. Généralement, le modèle est fourni sous la forme de deux fichiers correspondant respectivement à ces arcades, et comportant des données permettant de positionner les modèles de ces arcades l'un par rapport à l'autre dans la position d'occlusion.

Dans un mode de réalisation, le modèle de scène dentaire représente les deux arcades et on détermine un plan d'occlusion suivant les opérations suivantes :
I. détermination des points du modèle de scène dentaire qui appartiennent à une arcade et qui sont à une distance de l'autre arcade qui est inférieure à une distance prédéterminée de l'autre arcade, dits « points de contact » ;
II. optionnellement, filtrage d'une partie des points de contact, de préférence de manière que le nombre de points de contact appartenant à l'arcade maxillaire soit identique au nombre de points de contact appartenant à l'arcade mandibulaire, de préférence en éliminant les points d'une arcade les plus éloignés de l'autre arcade ;
III. régression linéaire, de préférence par la méthode des moindres carrés, sur l'ensemble des points de contact restant de manière à déterminer le plan d'occlusion.

A l'étape I., pour estimer les points de contact entre les dents des arcades maxillaire et mandibulaire (figure 6A), on détermine de préférence l'ensemble des points du modèle de l'arcade maxillaire et de l'arcade mandibulaire qui sont à une distance inférieure à une limite prédéterminée, cette limite étant de préférence inférieure à 3 mm, de préférence d'environ 2 mm. Tous les autres points de ces modèles sont ensuite ignorés, ce qui conduit à la représentation de la figure 6B.

A l'étape II., on supprime des points de contact de l'arcade qui en comporte le plus, jusqu'à ce que le nombre de points de contact soit identique pour les deux arcades. La précision de la régression linéaire à l'étape III. en est améliorée.

A l'étape III., une régression linéaire conventionnelle permet alors de déterminer le plan d'occlusion (référencé « P » sur la figure 6C).

Le modèle de scène dentaire peut ainsi être orienté en modifiant l'orientation du plan d'occlusion (Fig. 6D), par exemple en disposant le plan d'occlusion P à l'horizontal si le plan de référence P' est horizontal.

Si le modèle de scène dentaire ne comporte pas de données permettant de positionner les arcades maxillaire et mandibulaire l'une par rapport à l'autre, on utilise de préférence un mordu d'occlusion faisant apparaître les points de contact entre les dents supérieures de l'arcade maxillaire et les dents inférieures de l'arcade mandibulaire, puis on repositionne les modèles des arcades par rapport à ce mordu d'occlusion.

Pour déterminer l'orientation du modèle de scène dans le plan d'occlusion, il est nécessaire d'orienter l'axe longitudinal médian L avec un axe de référence L' du plan d'occlusion.

De préférence, on effectue les opérations suivantes :
i. projection, dans le plan d'occlusion du modèle de scène dentaire, des points de contact entre les dents des arcades maxillaire et mandibulaire du patient, les points de contact et/ou le plan d'occlusion étant de préférence déterminés suivant les étapes I à III ;
ii. détermination du barycentre des projections desdits points de contact et création d'un référentiel, dans le plan d'occlusion, centré sur ledit barycentre ;
iii. détermination, dans ledit référentiel, de la fonction parabolique, présentant le plus grand coefficient de corrélation avec l'ensemble des projections des points de contact ;
iv. rotation de l'ensemble des projections des points de contact autour du barycentre, et reprise de l'opération précédente *iii* jusqu'à ce que l'ensemble des projections des points de contact ait parcouru un secteur déterminé, de préférence supérieur à 90°, supérieur à 180°, voire d'environ 360° ;
v. identification du coefficient de corrélation le plus élevé pour l'ensemble des positions angulaires de l'ensemble des projections des points de contact autour du barycentre, et de l'axe de la fonction parabolique correspondante ;
vi. détermination de l'axe longitudinal médian du modèle de scène comme étant ledit axe de la fonction parabolique.

A l'étape ii., on considère des axes [Ox) et [Oy) dans le plan d'occlusion (figure 6C), le point O étant le barycentre des projections normales des points de contact sur le plan d'occlusion P.

A l'étape iii., on recherche dans ce référentiel (xOy), la courbe, de préférence parabolique, présentant le plus grand coefficient de corrélation avec l'ensemble desdites projections.

A l'étape iv., l'ensemble des projections des points de contact est ensuite déplacé dans le plan d'occlusion, par rotation autour du point O, et on recommence l'étape précédente à partir de cette nouvelle position angulaire des projections des points de contact.

Le cycle des opérations précédentes est poursuivi, de préférence jusqu'à avoir fait tourner l'ensemble des points de contact de 360° autour du barycentre O. On compare ensuite les coefficients de corrélation correspondant aux différentes orientations de l'ensemble des points de contact.

Aux étapes v. et vi., l'axe de la courbe qui conduit au coefficient de corrélation le plus élevé est considéré comme étant l'axe longitudinal médian (référencé « L » sur la figure 6C).

Le modèle de scène dentaire peut ainsi être orienté en modifiant l'orientation dans le plan d'occlusion (Fig. 6D), en alignant l'axe longitudinal médian L avec l'axe de référence L'. L'orientation « de base » du modèle de scène dans l'espace (figure 6D) peut être ainsi parfaitement déterminée, de manière rapide, par ordinateur.

Bien entendu, l'orientation dans l'espace du modèle de scène dentaire peut être réalisée par d'autres procédés, par exemple au moyen d'un réseau de neurones entrainé à cet effet.

**A l'étape 2)**, on détermine un ensemble constitué de différentes conditions d'observation du modèle de scène positionné dans ladite orientation de base, dites « conditions d'observation de base ». Cet ensemble est appelé « configuration d'observation ».

Pour acquérir une vue d'analyse de base, on positionne un appareil virtuel d'acquisition V dans des conditions d'observation de base respectives.

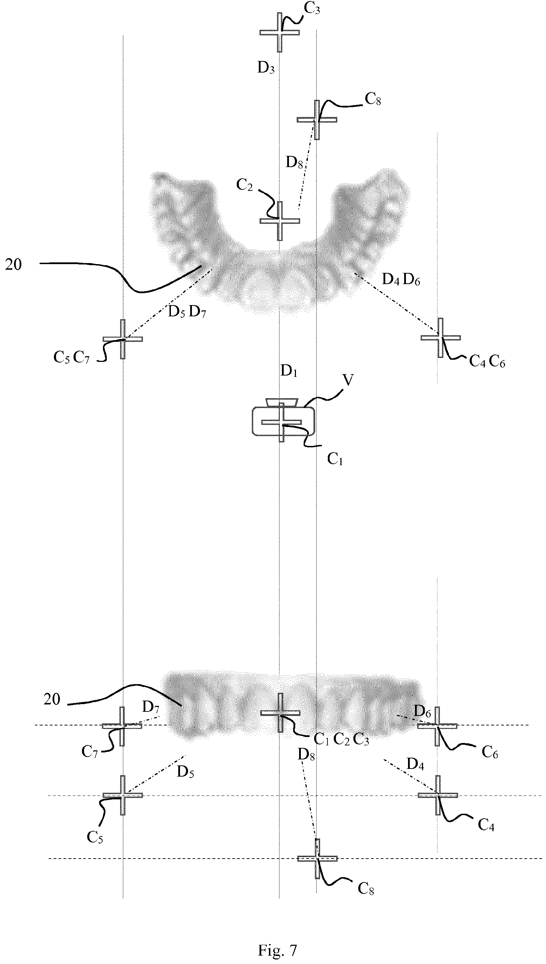

La figure 7 illustre un exemple d'une configuration d'observation comportant 8 conditions d'observation de base d'un modèle de scène 20. A chaque condition d'observation de base sont associées
- une position d'observation Cᵢ différente, représentée par une croix, et
- une direction d'observation du modèle de scène, Dᵢ, pour l'appareil d'acquisition
virtuel V destiné à l'acquisition de la vue d'analyse de base correspondante, i étant compris entre 1 et 8.

La détermination de la configuration d'observation peut être effectuée manuellement, en testant différentes configurations d'observation.

De préférence, la configuration d'observation est déterminée de manière à minimiser le nombre de conditions d'observation de base. De préférence, on minimise le recouvrement entre les différentes vues d'analyse de base obtenues avec la configuration d'observation. Autrement dit, la configuration d'observation est déterminée pour que la surface du modèle de scène représentée sur plus d'une vue d'analyse de base soit minimale.

La configuration d'observation est de préférence déterminée en fonction des modèles élémentaires à créer. Par exemple, pour modéliser les dents, il est préférable que la configuration d'observation ne contienne pas de vue d'analyse qui ne représente pas au moins dent.

On appelle « degré de précision » le pourcentage de la surface du modèle de scène qui, à l'issue de l'étape e) de l'étape 4), a été affectée à un modèle élémentaire. Il est en effet possible que l'ensemble des régions du modèle de scène représentées par des zones élémentaires sur les différentes vues d'analyse de base utilisées ne suffise pas couvrir toute la surface du modèle de scène.

De préférence, à l'étape 2), on détermine la configuration d'observation en fonction d'un degré de précision minimal à atteindre pour les modèles élémentaires. De préférence, la configuration d'observation est déterminée de manière que le degré de précision soit supérieur à 90%, de préférence supérieur 95%, voire supérieur à 98%.

De préférence, à l'étape 2), on détermine la configuration d'observation de manière à minimiser le nombre de conditions d'observation de base, tout en obtenant des modèles élémentaires ayant un degré de précision supérieur à un degré de précision minimal.

**A l'étape 3)**, on acquiert les vues d'analyse, dites « vues d'analyse de base », en observant le modèle de scène dentaire dans les conditions d'observation de base.

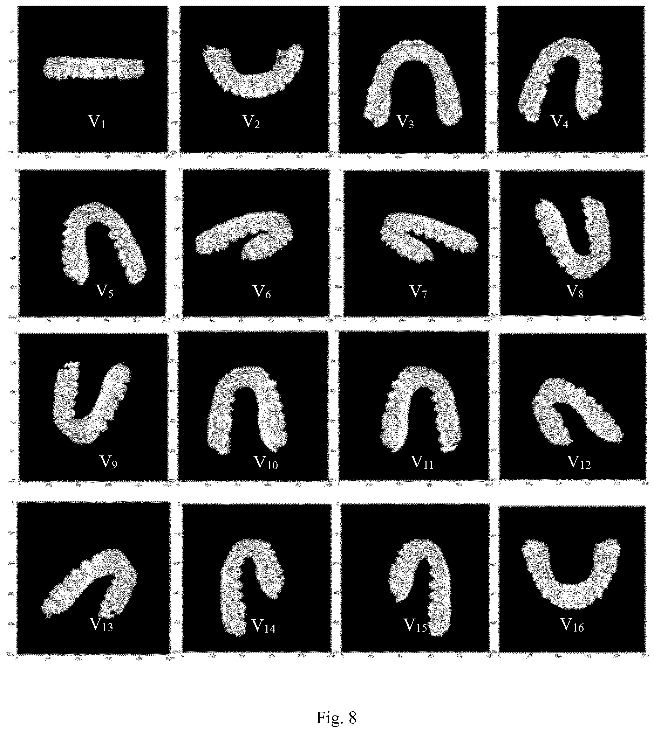

La figure 8 illustre un ensemble de vues d'analyse de base Vᵢ, i étant compris entre 1 et 16, correspondant à un exemple de configuration d'observation. Les 8 premières vues d'analyse de base Vᵢ correspondent sensiblement aux conditions d'observation de base illustrées sur la figure 7.

L'étape 3) peut être mise en oeuvre complètement avant l'étape 4), ou progressivement lors de la mise en oeuvre du procédé de découpage selon l'invention, à mesure que les vues d'analyse de base sont utilisées.

**A l'étape 4),** on utilise les vues d'analyse de base pour la mise en oeuvre du procédé de découpage selon l'invention.

Par exemple, à l'étape a), on utilise la vue d'analyse de base Vi, puis on effectue 7 cycles d'étapes a) à d) avec les vues d'analyse de base V₂ à Vs, puis on effectue une étape e).

De manière remarquable, un découpage de bonne qualité est possible avec un nombre de vues d'analyse de base limité, en particulier avec moins de 50, moins de 30, moins de 20 ou même moins de 15 vues d'analyse de base. Le procédé de découpage est ainsi considérablement accéléré. Le degré de précision reste cependant très élevé. Avec l'ensemble des vues d'analyse de base de la figure 8, il dépasse 98%.

De préférence, le procédé de découpage perfectionné comporte encore, à l'issue de l'étape 4), l'étape suivante :
5) affectation de chacun d'au moins une partie des voxels orphelins du modèle de scène, de préférence de chacun des voxels orphelins, aux modèles élémentaires créés à l'étape e), un voxel orphelin ne pouvant être affecté à plus d'un modèle élémentaire.

Un voxel « orphelin » est un voxel non regroupé au sein d'un modèle élémentaire lors de l'étape e). Par exemple, si le degré de précision est de 95%, les voxels qui constituent les 5% complémentaires du modèle de scène sont des voxels orphelins.

Les règles d'affectation d'un voxel orphelin à un modèle élémentaire dépendent de l'objet modélisé par le modèle élémentaire. Par exemple, un voxel orphelin peut être affecté au modèle élémentaire le plus proche.

Selon l'invention, à l'étape 2), on détermine la configuration d'observation de manière à minimiser le nombre de conditions d'observation de base dans ladite configuration d'observation, tout en obtenant, à l'issue de l'étape 4), des modèles élémentaires ayant un degré de précision supérieur à un seuil prédéterminé, de préférence un degré de précision minimal, pour chaque modèle de scène « de base » d'un groupe comportant plus de 10, plus de 100, de préférence plus de 1 000 modèles de scène de base différents.

Avantageusement, la configuration d'observation ainsi déterminée est adaptée à de nombreux modèles de scène, dits « modèles de scène de base ». Si le groupe comporte un échantillon de modèles de scène de base représentatif des arcades dentaires d'une population, la configuration d'observation peut avantageusement convenir pour un modèle de scène quelconque, c'est-à-dire pour tout patient de cette population.

Bien entendu, l'invention n'est pas limitée aux modes de réalisation décrits ci-dessus et représentés.

En particulier, le patient n'est pas limité à un être humain. Un procédé selon l'invention peut être utilisé pour un autre animal.

## Revendications

1. Procédé de découpage d'un modèle tridimensionnel d'une scène dentaire, ou « modèle de scène », ledit procédé comportant les étapes suivantes :
a) acquisition d'une vue dudit modèle de scène, dite « vue d'analyse » ;
b) analyse de la vue d'analyse au moyen d'un réseau de neurones entrainé pour
- identifier, dans ladite vue d'analyse, au moins une zone élémentaire représentant un élément de la scène dentaire, et
- déterminer d'une valeur pour au moins un attribut de ladite zone élémentaire ;
c) identification d'une région du modèle de scène représentée par ladite zone élémentaire sur ladite vue d'analyse, et affectation, à ladite région, d'une valeur d'un attribut dudit modèle de scène, ou « attribut de modèle », ladite valeur de l'attribut de modèle étant dépendante de la valeur dudit attribut de la zone élémentaire,
d) modification de la vue d'analyse, puis reprise à l'étape b),
le procédé comportant, après plus de 10 cycles d'étapes b) à d), l'étape suivante :
e) regroupement des régions du modèle de scène qui ont la même valeur pour un attribut dudit modèle de scène, de manière à créer un modèle élémentaire
la vue d'analyse acquise à l'étape a) et/ou une ou plusieurs des vues d'analyse modifiées à l'étape d), de préférence la vue d'analyse acquise à l'étape a) et toutes les vues d'analyse modifiées à l'étape d), étant des vues d'analyse de base obtenues suivant les étapes suivantes :
1) détermination d'une orientation prédéterminée du modèle de scène, dite « orientation de base » ;
2) détermination d'un ensemble de différentes conditions d'observation du modèle de scène positionné dans ladite orientation de base, dites « conditions d'observation de base » ;
3) acquisition d'un ensemble de vues d'analyse de base, chaque vue d'analyse de base étant acquise dans des conditions d'observation de base respectives
procédé dans lequel, à l'étape 2), on détermine l'ensemble desdites conditions d'observation de base de manière à minimiser le nombre desdites conditions d'observation de base, et à maintenir à une valeur supérieure à un seuil prédéterminé, pour chaque modèle de scène de base d'un groupe de modèles de scène de base, le rapport de la surface totale de l'ensemble des modèles élémentaires déterminés à l'étape e) sur la surface du modèle de scène.

2. Procédé selon la revendication immédiatement précédente, dans lequel ledit groupe de modèles de scène de base comporte plus de 10, de préférence plus de 100, de préférence plus de 1 000 modèles de scène de base, chaque modèle de scène de base modélisant au moins une partie d'une arcade d'un patient respectif.

3. Procédé selon l'une quelconque des revendications précédentes, comportant, à l'issue de l'étape e), l'étape consistant à affecter chacun d'au moins une partie des voxels orphelins du modèle de scène, de préférence de chacun des voxels orphelins, aux modèles élémentaires créés à l'étape e), un voxel orphelin ne pouvant être affecté à plus d'un modèle élémentaire, un voxel orphelin étant un voxel non regroupé au sein d'un modèle élémentaire lors de l'étape e).

4. Procédé selon l'une quelconque des revendications précédentes, dans lequel, à l'étape c), l'attribut dudit modèle de scène est identique à l'attribut de la zone élémentaire.

5. Procédé selon l'une quelconque des revendications précédentes, dans lequel, à l'étape b), l'attribut de la zone élémentaire est choisi parmi un numéro de dent, un type de dent, un paramètre de forme de la dent, un indice de déflexion en mésial et distal du bord incisif, ou un niveau d'abrasion, un paramètre d'apparence de la dent, un paramètre relatif à l'état de la dent et un paramètre relatif à un appareil dentaire porté par la dent.

6. Procédé selon la revendication immédiatement précédente, dans lequel l'attribut de la zone élémentaire est choisi parmi un numéro de dent et un type de dent.

## Patentansprüche

1. Verfahren zum Ausschneiden eines dreidimensionalen Modells einer Zahnszene, oder "Szenemodell", wobei das Verfahren die folgenden Schritte aufweist:
a) Erfassung einer Ansicht des Szenemodells, genannt "Analyseansicht";
b) Analyse der Analyseansicht mittels eines neuronalen Netzwerks, das trainiert wird, um
- in der Analyseansicht mindestens eine Elementarzone zu identifizieren, die ein Element der Zahnszene darstellt, und
- über einen Wert für mindestens ein Attribut der Elementarzone zu bestimmen;
c) Identifizierung einer Region des Szenemodells, die von der Elementarzone auf der Analyseansicht dargestellt wird, und Zuordnung, zu der Region, eines Werts eines Attributs des Szenemodells, oder "Modellattribut", wobei der Wert des Modellattributs vom Wert des Attributs der Elementarzone abhängt,
d) Änderung der Analyseansicht, dann Wiederbeginn im Schritt b),
wobei das Verfahren nach mehr als 10 Zyklen von Schritten b) bis d) den folgenden Schritt aufweist:
e) Gruppierung der Regionen des Szenemodells, die den gleichen Wert für ein Attribut des Szenemodells haben, um ein Elementarmodell zu erzeugen,
wobei die im Schritt a) erfasste Analyseansicht und/oder eine oder mehrere der im Schritt d) geänderten Analyseansichten, vorzugsweise die im Schritt a) erfasste Analyseansicht, und alle im Schritt d) geänderten Analyseansichten Basisanalyseansichten sind, die gemäß den folgenden Schritten erhalten werden:
1) Bestimmung einer vorbestimmten Ausrichtung des Szenemodells, "Basisausrichtung" genannt;
2) Bestimmung einer Einheit von verschiedenen Beobachtungsbedingungen des in der Basisausrichtung positionierten Szenemodells, "Basisbeobachtungsbedingungen" genannt;
3) Erfassung einer Einheit von Basisanalyseansichten, wobei jede Basisanalyseansicht unter Basisbeobachtungsbedingungen erfasst wird,
Verfahren, bei dem im Schritt 2) die Einheit der Basisbeobachtungsbedingungen bestimmt wird, um die Anzahl der Basisbeobachtungsbedingungen zu minimieren und für jedes Basisszenemodell einer Gruppe von Basisszenemodellen das Verhältnis der Gesamtfläche der Einheit der im Schritt e) bestimmten Elementarmodelle zur Fläche des Szenemodells auf einem Wert höher als eine vorbestimmte Schwelle zu halten.

2. Verfahren nach dem unmittelbar vorhergehenden Anspruch, wobei die Gruppe von Basisszenzemodellen mehr als 10, vorzugsweise mehr als 100, vorzugsweise mehr als 1 000 Basisszenemodelle aufweist, wobei jedes Basisszenemodell mindestens eine Teil eines Zahnbogens eines Patienten modelliert.

3. Verfahren nach einem der vorhergehenden Ansprüche, das nach dem Schritt e) den Schritt aufweist, der darin besteht, jedes von mindestens einem Teil der verwaisten Voxel des Szenemodells, vorzugsweise von jedem der verwaisten Voxel, den im Schritt e) erzeugten Elementarmodellen zuzuordnen, wobei ein verwaistes Voxel nicht mehr als einem Elementarmodell zugeordnet werden kann, wobei ein verwaistes Voxel ein nicht im Schritt e) innerhalb eines Elementarmodells gruppiertes Voxel ist.

4. Verfahren nach einem der vorhergehenden Ansprüche, wobei im Schritt c) das Attribut des Szenemodells gleich dem Attribut der Elementarzone ist.

5. Verfahren nach einem der vorhergehenden Ansprüche, wobei im Schritt b) das Attribut der Elementarzone ausgewählt wird unter einer Zahnnummer, einem Zahntyp, einem Formparameter des Zahns, einem mesialen und distalen Ablenkungsindex der Schneidekante oder einem Abrasionsgrad, einem Erscheinungsparameter des Zahns, einem Parameter bezüglich des Zustands des Zahns und einem Parameter bezüglich einer vom Zahn getragenen Zahnklammer.

6. Verfahren nach dem unmittelbar vorhergehenden Anspruch, wobei das Attribut der Elementarzone unter einer Zahnnummer und einem Zahntyp ausgewählt wird.

## Claims

1. Method for cutting a three-dimensional model of a dental scene, or "scene model", said method comprising the following steps:
a) acquiring a view of said scene model, called the "analysis view";
b) analysing the analysis view by means of a trained neural network in order to
- identify, in said analysis view, at least one elementary zone representing an element of the dental scene, and
- determine a value for at least one attribute of said elementary zone;
c) identifying a region of the scene model represented by said elementary zone on said analysis view, and assigning, to said region, a value of an attribute of said scene model, or "model attribute", said value of the model attribute being dependent on the value of said attribute of the elementary zone,
d) modifying the analysis view, then resuming at step b),
the method comprising, after more than 10 cycles of steps b) to d), the following step:
e) grouping the regions of the scene model which have the same value for an attribute of said scene model, so as to create an elementary model,
the analysis view acquired at step a) and/or one or more of the analysis views modified at step d), preferably the analysis view acquired at step a) and all the analysis views modified at step d), being base analysis views obtained according to the following steps:
1) determining a predetermined orientation of the scene model, called the "base orientation";
2) determining a set of different conditions of observation of the scene model positioned in said base orientation, called "base observation conditions";
3) acquiring a set of base analysis views, each base analysis view being acquired under respective base observation conditions,
in which method, at step 2), the set of said base observation conditions is determined in such a way as to minimize the number of said base observation conditions and to maintain, at a value greater than a predetermined threshold, for each base scene model of a group of base scene models, the ratio of the total surface area of the set of the elementary models, determined at step e), to the surface area of the scene model.

2. Method according to the immediately preceding claim, in which said group of base scene models comprises more than 10, preferably more than 100, preferably more than 1,000 base scene models, each base scene model modelling at least part of a dental arch of a respective patient.

3. Method according to either of the preceding claims, comprising, at the end of step e), the step of assigning each of at least some of the orphan voxels of the scene model, preferably each of the orphan voxels, to the elementary models created at step e), an orphan voxel not being able to be assigned to more than one elementary model, an orphan voxel being a voxel that is not grouped within an elementary model at step e).

4. Method according to any one of the preceding claims, in which, at step c), the attribute of said scene model is identical to the attribute of the elementary zone.

5. Method according to any one of the preceding claims, in which, at step b), the attribute of the elementary zone is chosen from a tooth number, a tooth type, a tooth shape parameter, an index of mesial and distal deflection of the incisal margin, or a level of abrasion, a tooth appearance parameter, a parameter relating to the state of the tooth, and a parameter relating to a dental appliance carried by the tooth.

6. Method according to the immediately preceding claim, in which the attribute of the elementary zone is chosen from a tooth number and a tooth type.
